Europäisches Patentamt

European Patent Office

Office européen des brevets

(11) Publication number: **0 382 875**
**A1**

# EUROPEAN PATENT APPLICATION

(21) Application number: 89102769.0

(51) Int. Cl.⁵: **A61K 47/28, A61K 37/00**

(22) Date of filing: 17.02.89

(43) Date of publication of application:
**22.08.90 Bulletin 90/34**

(84) Designated Contracting States:
**DE FR GB IT**

(71) Applicant: **Matuo, Yuhsy**
**640-9, Hiragi Miki-cho**
**Kita-gun Kagawa-ken(JP)**
(84) **DE FR GB IT**

Applicant: **ORIENTAL YEAST CO., LTD.**
**10-gou, 6-ban, 3-chome, Azusawa Itabashi-ku**
**Tokyo 174(JP)**
(84) **DE**

(72) Inventor: **Matuo, Yuhsy**

640-9, Hiragi, Miki-cho
Kita-gun, Kagawa-ken(JP)
Inventor: **Nishi, Nozomu**
A-208, 505-2, Maedahigashi-machi
Takamatsu-shi, Kagawa-ken(JP)
Inventor: **Wada, Fumio**
D-203, 505-2, Maedahigashi-machi
Takamatsu-shi, Kagawa-ken(JP)
Inventor: **Takagahara, Isamu**
3-59, 5-chome, Fushimidai, Inagawa-cho
Kawabe-gun, Hyogo-ken(JP)

(74) Representative: **Thomsen, Dieter, Dr.rer.nat.**
**Kaiser-Ludwig-Platz 6**
**D-8000 München 2(DE)**

(54) **Dispersant for biologically active peptide.**

(57) The present invention relates to a dispersant comprising as an effective ingredient 3-[(3-cholamidopropyl) dimethylammonio]-1-propane sulfonate (hereafter referred to as CHAPS) or 3-[(3-cholamidopropyl)-dimethylammonio]-2-hydroxy-1-propane sulfonate (hereafter referred to as CHAPSO) that is useful for stabilization of biologically active (poly)peptides.

FIG. 1

## DISPERSANT FOR BIOLOGICALLY ACTIVE PEPTIDE

Field of the Invention

The present invention relates to a dispersant comprising as an effective ingredient 3- [(3-cholamidopropyl) dimethylammonio]-1-propane sulfonate (hereafter referred to as CHAPS) or 3-(3-cholamidopropyl)dimethylammonio]-2-hydroxy-1-propane sulfonate (hereafter referred to as CHAPSO) that is useful for stabilization of biologically active (poly)peptides.

In the present invention, the presence of CHAPS or CHAPSO in an extremely trace amount can prevent biologically active (poly)peptides from being irreversiblly adsorbed onto the surface of a container, an equipment or a carrier for chromatography in biochemical manipulations, etc. and thus from not participating as active molecules in the manipulations or from not being purified. Therefore, the present invention greatly contributes to life science such as biochemistry and pharmacology.

Problems to be solved by the Invention and the Prior Art

In general, biologically active factors such as FGF (fibroblast growth factor) and the like are composed of (poly)peptide and exhibit their activities in a trace amount. Accordingly, these biologically active (poly)-peptides are generally used for reaction or test in a markedly low concentration.

Biologically active (poly)peptides used in such a markedly low concentration are adsorbed onto the surface of a container or an equipment used for the reaction or test and fail to participate as active forms.

In case that biologically active (poly)peptides are adsorbed onto the surface of a container or equipment, a (poly)peptide concentration in the reaction changes so that a problem occurs that accurate measurement results cannot be obtained.

Thus, extraneous protein such as serum albumin which is known inert to culture cells and can give a standard having a relatively high purity have been chosen and added to a solution containing bilogically active (poly)peptides in the prior art.

However, the addition of serum albumin, etc. involves defects of causing hindrance in examining physicochemical properties of biologically active factors per se and greatly reducing purity upon preparation of biologically active (poly)peptide products, even though its stabilizing action of biologically active (poly)-peptides is recognized.

Brief Description of the Drawings

Fig. 1 shows cytotoxicity of each dispersant used in Example 1.
Fig. 2 shows cytotoxicity of CHAPS in Example 2.

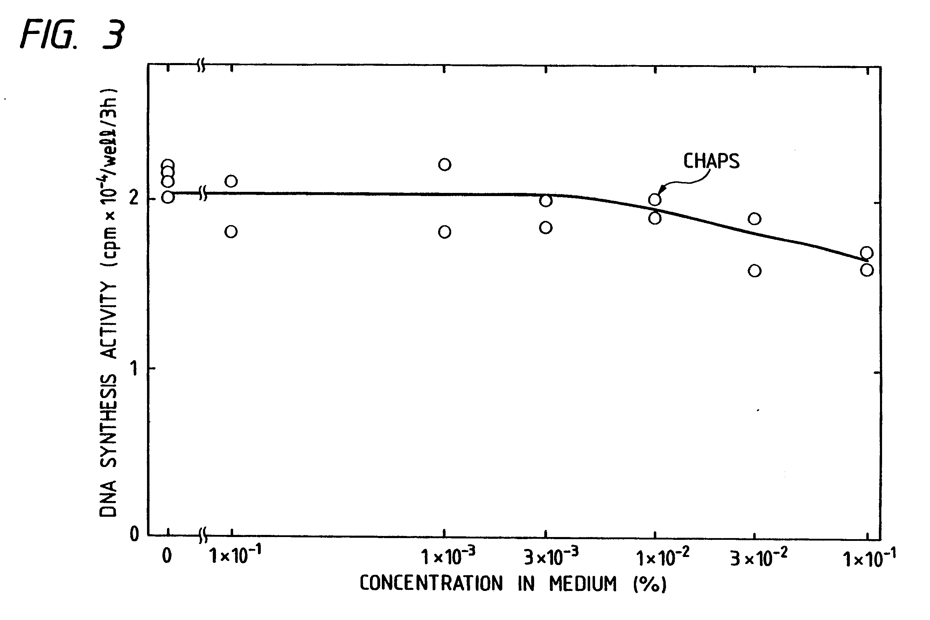

Fig. 3 shows cytotoxicity of CHAPS in Example 3.
Fig. 4 shows preventive effect of CHAPS on FGF at each concentration in Example 6.
Fig. 5 shows influence on FGF stability at 50°C when added with CHAPS or without CHAPS in Example 8.
Fig. 6 shows influence of CHAPS and NaCl concentrations on molecular-sieve chromatography in Example 9 when added with CHAPS or without CHAPS.
Fig. 7 shows HPLC of FGF when chromatographed without CHAPS.
Fig. 8 shows HPLC of FGF when chromatographed with CHAPS.

Means for solving the Problems

The present inventors have made extensive investigation to develop dispersants for bilogically active (poly)peptides which have properties quite different from those of other biologically active substances, have minimized toxicity to cultured cells and are more excellent. As a result, it has been discovered that the problems have been solved by using CHAPS or CHAPSO.

The present invention relates to a dispersant for comprising as an effective ingredient CHAPS or CHAPSO that is useful for stabilization of biologically active (poly)peptides.

CHAPS or CHAPSO is a kind of zwitterionic surface active agents and known. It is quite unknown, however, that CHAPS or CHAPSO is useful as stabilizing dispersants for biologically active (poly)peptides.

Sufficient amount of CHAPS or CHAPSO to be added is in the range of 0.05 to 1.0%, preferably about 0.1%, based on liquid containing biologically active (poly)peptides.

Biologically active (poly)peptides in a solution added with CHAPS or CHAPSO are well dispersed so that adsorption onto the surface of a container or measurement devices is markedly reduced. For example, in applying a biologically active (poly)peptides solution to various chromatographic columns, the addition of CHAPS or CHAPSO results in a remarkably high recovery. Further by adding CHAPS or CHAPSO upon dialysis or filtration of biologically active (poly)peptides, adsorption onto the membranes can be prevented almost perfectly.

Furthermore, by the addition of CHAPS or CHAPSO, biologically active (poly)peptides in solution can be stably stored at low temperatures or in a frozen state over long periods of time.

The thus added CHAPS or CHAPSO can be removed, depending upon necessity and such is advantageous.

That is, CHAPS or CHAPSO can be removed by utilizing difference in solubility in organic solvents. CHAPS or CHAPSO is soluble in water, methanol and dimethylsulfoxide but insoluble in other solvents (acetone, acetonitrile, ether, chloroform, 2-methyl-1-propanol, N,N-dimethylformamide, isoamly alcohol, ethanol, hexane, xylene, petroleum ether, benzene, n-heptane, toluene). Utilizing the difference in solubility, CHAPS or CHAPSO can easily be removed.

In biologically active (poly)peptides adsorbed to heparin-Sepharose, CHAPS or CHAPSO which is not adsorbed thereto can be removed utilizing such a difference. Alternatively, CHAPS or CHAPSO can also be removed by reversed phase HPLC such as C4 column.

Next, examples of the present invention are shown. DNA synthesis activity or FGF activity of samples was determined by the method shown in Example 1 and expressed by cpm (counts per minute) x $10^{-4}$/well/3h.

## Example 1

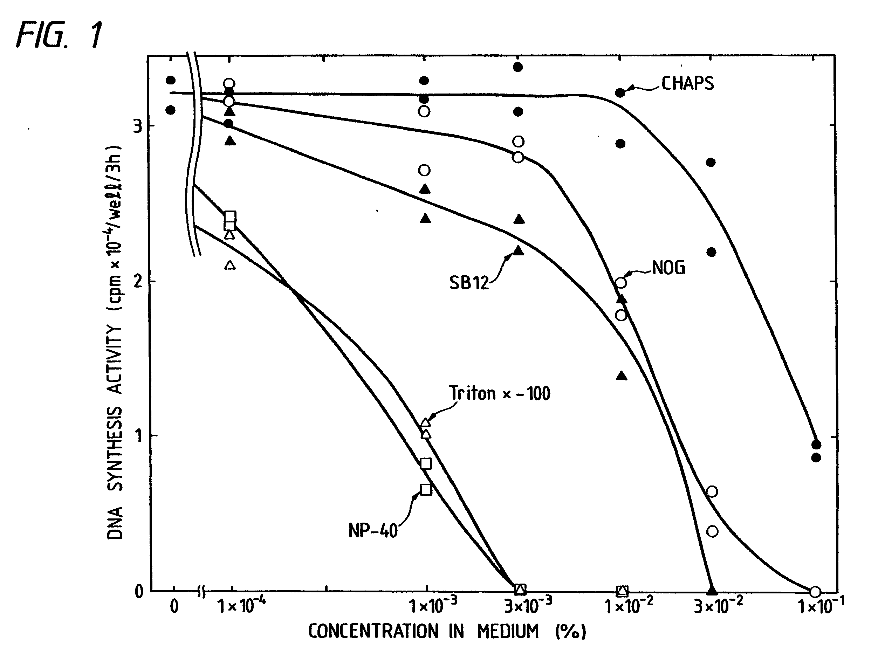

Upon culturing BALB/C3T3 fibroblasts in bovine serum-supplemented medium on a 24 well plate, CHAPS, NOG (1-O-n-octyl-$\beta$-D-glucopyranoside), SB12 (3-(dodecylmethylammonio)-1-propane sulfonate: sulfobetaine 12), Triton X-100 and NP-40 (Nonidet P-40) were added to the medium at concentrations of 1 x $10^{-4}$ to 1 x $10^{-1}$%. After culturing for 19 hours, DNA synthesis activity was measured and the results shown in Fig. 1 were obtained.

From Fig. 1, it is understood that CHAPS which is a kind of zwitterionic surface active agents shows the least cytotoxicity among various surface active agents.

Using CHAPSO, similar experiments were carried out and similar results were obtained.

## Method

BALB/C3T3 cells, a kind of fibroblasts established from a mouse fetus were suspended in 3% bovine serum-containing DME medium (DME-3% CS) at a concentration of 2 x $10^4$/ml. One milliliter of the suspension was taken and cultured at 37° C for 5 hours on a 24 well tissue culture dish. Then, the medium was replaced by 1 ml of DME-1% CS followed by culturing at 37° C for 48 hours. A sample (2 to 20 $\mu$l) was added thereto, followed by culturing for further 16 hours.

Then, 10 $\mu$l of 2 x $10^{-5}$ M[$^3$H]thymidine (40 $\mu$Ci/ml) was supplemented. After culturing for 3 hours, radioactivity incorporated into DNA fraction was measured. That is, after the medium was removed, cells were rinsed with 1 ml of PBS (phosphate-buffered saline) once and 3 times with 1 ml of cold 10% TCA (trichloroacetic acid). Then, 1 ml of 0.5 M NaOH was added thereto. After allowing to stand at room temperature for 15 minutes, the mixture was neutralized with 5 M HCl. Scintillator cocktail, 6 ml, was added thereto to measure the radioactivity.

## Example 2

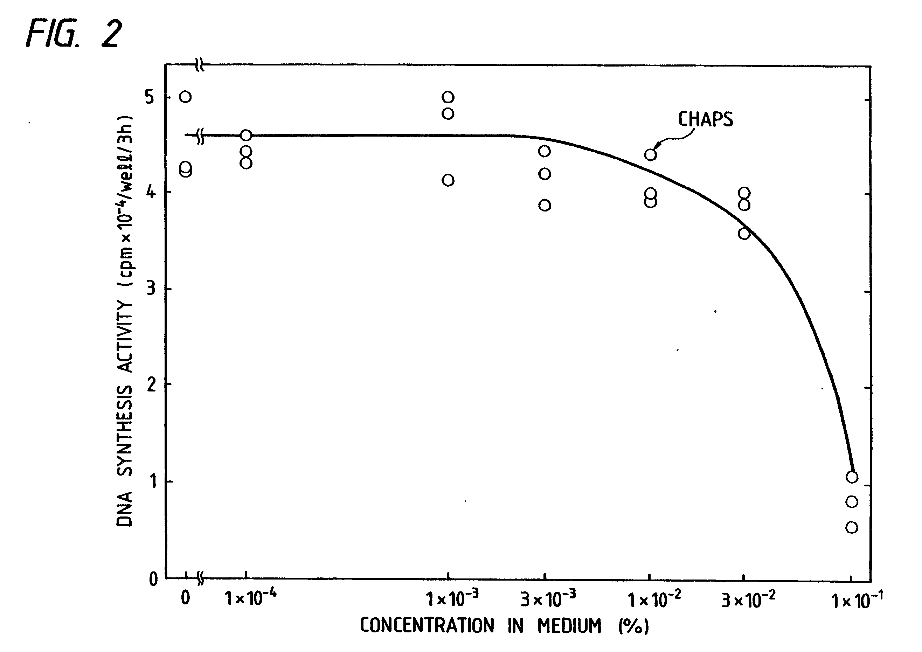

AT-3 prostatic adenocarcinoma cells were cultured in a manner similar to Example 1 by adding CHAPS thereto except that DME/F-12 medium was used. FGF activity was measured 19 hours after and the results shown in Fig. 2 were obtained.

Using CHAPSO, similar results were obtained.

### Example 3

MC 3T3-El osteoblasts were cultured in a manner to Example 1 by adding CHAPS thereto except that $\alpha$-MEM medium was used. FGF activity was measured 19 hours after and the results shown in Fig. 1 were obtained.

### Example 4

Commercially available FGF (fibroblast growth factor) was charged in containers shown in Table 1, respectively, in 0.5 $\mu$g/ml PBS; the containers were charged with or without 0.1% CHAPS. After storing at 4°C for 2 days, FGF activity in each container was measured. The results shown in Table 1 were obtained.

Table 1

| Adsorption of FGF to Containers: Kind of Container and Stabilization with CHAPS | | |
|---|---|---|
| Kind of Container | FGF Activity - CHAPS | (cpm x $10^{-3}$/well/3h) +0.1% CHAPS |
| Test tube made of glass | 4.3 | 23.4 |
| Silicon-treated test tube made of glass | 2.3 | 16.1 |
| Minisorp test tube (made of polyethylene) | 15.7 | 31.6 |
| Plastic test tube (made of polystyrene) | 2.5 | 24.0 |

From Table 1, it is understood that adsorption of FGF onto the containers can be effectively prevented by addition of 0.1% CHAPS.

### Example 4

Commercially available FGF was charged in containers made of glass at concentrations (PBS) shown in Table 2, respectively; all of the containers were not charged with 0.1% CHAPS. After storing at 4°C for 2 days, FGF activity in each container was measured. The results shown in Table 2 were obtained. The activity measurement was all performed in a concentration of 10 ng/ml.

Table 2

| Difference in Adsorption of FGF to Glass Containers at Various Concentrations | |
|---|---|
| Concentration of FGF | FGF Activity (cpm x $10^{-4}$/well/3h) |
| 10 $\mu$g/ml | 16.8 |
| 5 $\mu$g/ml | 2.5 |
| 1 $\mu$g/ml | 1.1 |
| 0.5 $\mu$g/ml | 0.7 |

From the results shown in Figs. 1 and 2, it is understood that the loss due to adsorption occurs in the range of 30 to 40% even at a concentration of FGF as high as 10 $\mu$g/ml when 0.1% CHAPS was not present.

4

Example 5

Commercially available FGF was dialyzed at 4°C in a concentration of 0.5 μg/ml (PBS) in the presence of CHAPS at concentrations shown in Table 3. After dialysis, CHAPS (0.1%) was added to all of the samples and the samples were transferred to polyethylene containers. FGF activity in each container was determined and the results shown in Table 3 were obtained. As a dialysis membrane, SPECTRAPOR (3,500 cut off) was used.

Table 3

| Loss Caused During Dialysis of FGF and Stabilization by CHAPS | |
| --- | --- |
| CHAPS (%) | FGF Activity (cpm x $10^{-4}$/well/3h) |
| 0 | 9.2 |
| 0.01 | 33.6 |
| 0.1 | 38.6 |

Example 6

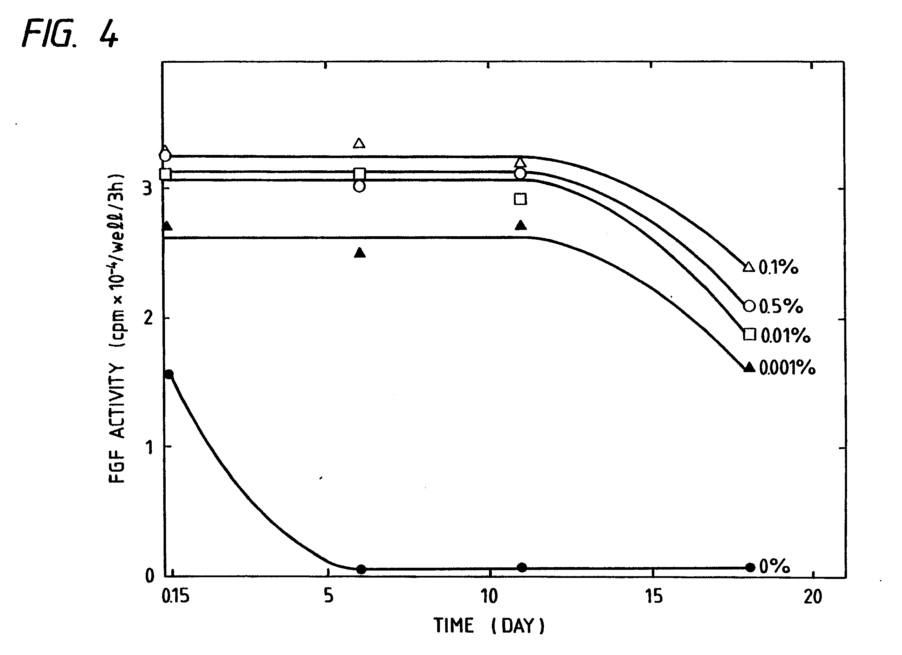

Commercially available FGF was charged in containers made of glass at a concentration of 0.5 μg/ml (PBS) by adding CHAPS at concentrations shown in Fig. 4, respectively. After storing at 4°C for 18 days, FGF activity in each container was measured and expressed by cpm x $10^{-4}$/well/3h. The results shown in Fig. 4 were obtained.

From Fig. 4, it is understood that owing to the addition of 0.1% CHAPS, FGF of 0.5 μg/ml concentration can be stored stably at 4°C for 11 days.

Using CHAPSO, similar results were obtained.

Example 7

Commercially available FGF (0.5 μg/ml) was stored at -20°C in containers made of glass in the presence of CHAPS at various concentrations. Samples were melted every time when they were subjected to activity assay, and FGF activity was measured with an aliquot of the melt.

The results are shown in Table 4.

Table 4

| Stabilizing Effect of CHAPS on Storage of FGF at -20°C | | | |
|---|---|---|---|
| Date at -20°C | FGF Activity (cpm x $10^{-4}$/well/3h) + CHAPS (%) | | |
| | 0 | 0.001 | 0.01 | 0.1 |
| 0 | 15.1 | 24.1 | 27.5 | 25.9 |
| | 14.3 | 25.3 | 30.0 | 28.7 |
| 7 | 6.5 | 29.1 | 35.0 | 26.3 |
| | 6.4 | 27.0 | 30.3 | 23.8 |
| 20 | 0.3 | 30.1 | 31.3 | 29.3 |
| | 0.5 | 29.3 | 36.4 | 23.4 |
| 40 | 0.6 | 18.5 | 23.5 | 24.8 |
| | 0.8 | 16.6 | 22.7 | 27.2 |

## Example 8

After commercially available FGF (5 μg/ml) added with 1% CHAPS or added with no CHAPS was allowed to stand at 50°C for 1 hour, stability of FGF was examined. FGF activity was expressed by cpm x $10^{-4}$/well/3h.

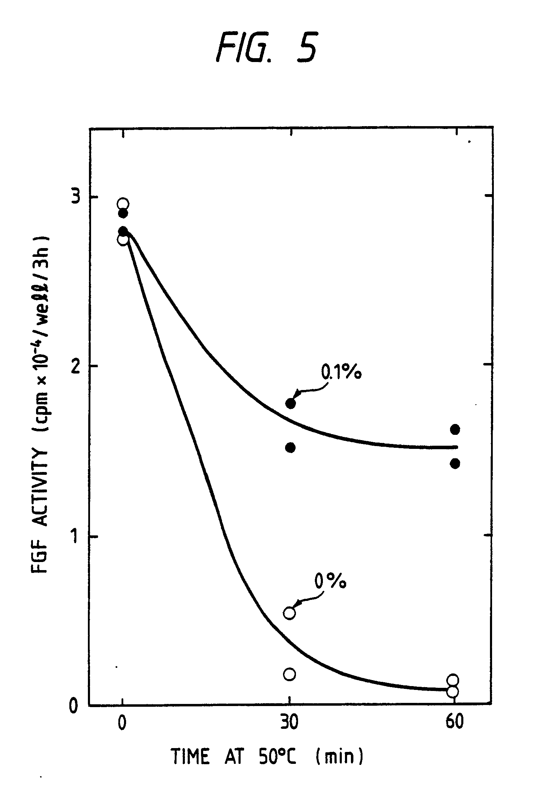

The results are shown in Fig. 5. From Fig. 5, it is understood that CHAPS shows stabilizing effect also on thermal instability of FGF.

## Example 9

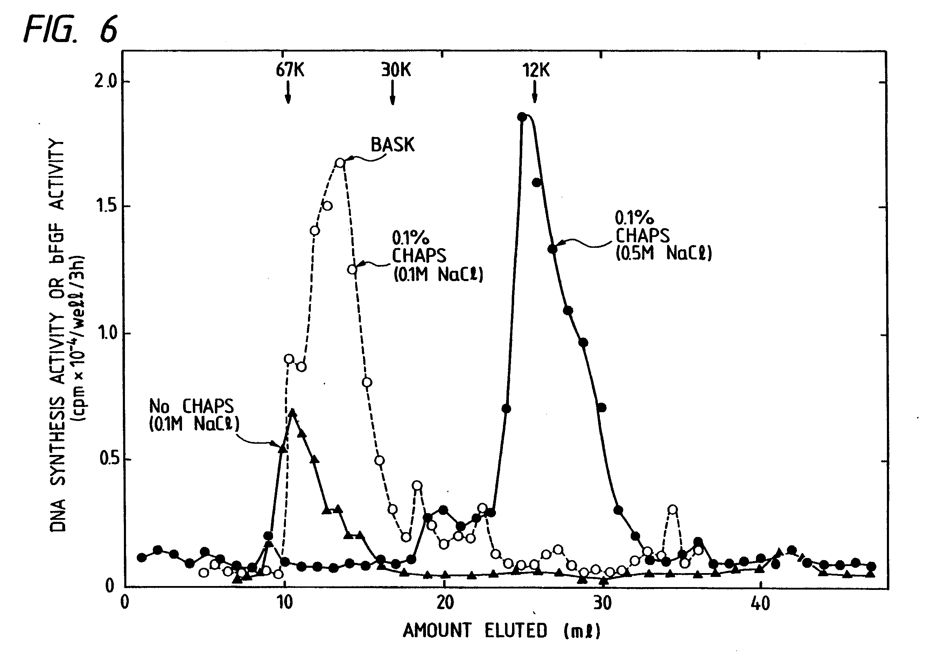

Comparison of crude basic FGF (bFGF) in molecular-sieve chromatography was made between addition with 0.1% CHAPS and addition with no CHAPS. The results are shown in Fig. 6.

From Fig. 6, it is understood that when no CHAPS was added, bFGF was adsorbed onto a carrier and device for chromatography in considerable amounts.

## Example 10

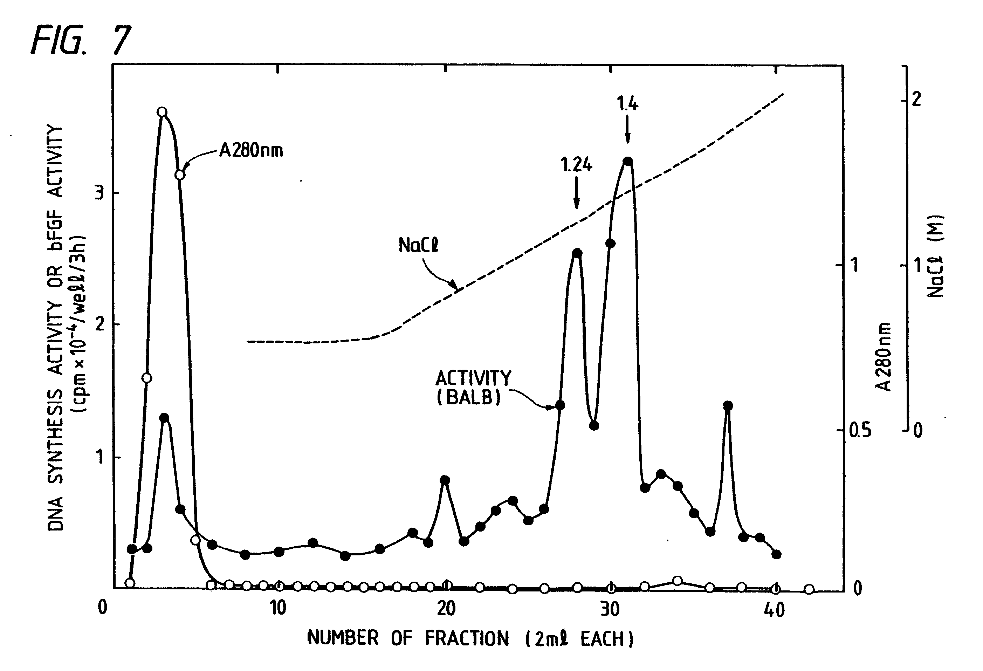

An extract from human benign prostatic hypertrophy (BPH) tissues was subjected to Heparin-5PW HPLC without adding any CHAPS thereto. Elution curves of FGF activity are shown in Fig. 7.

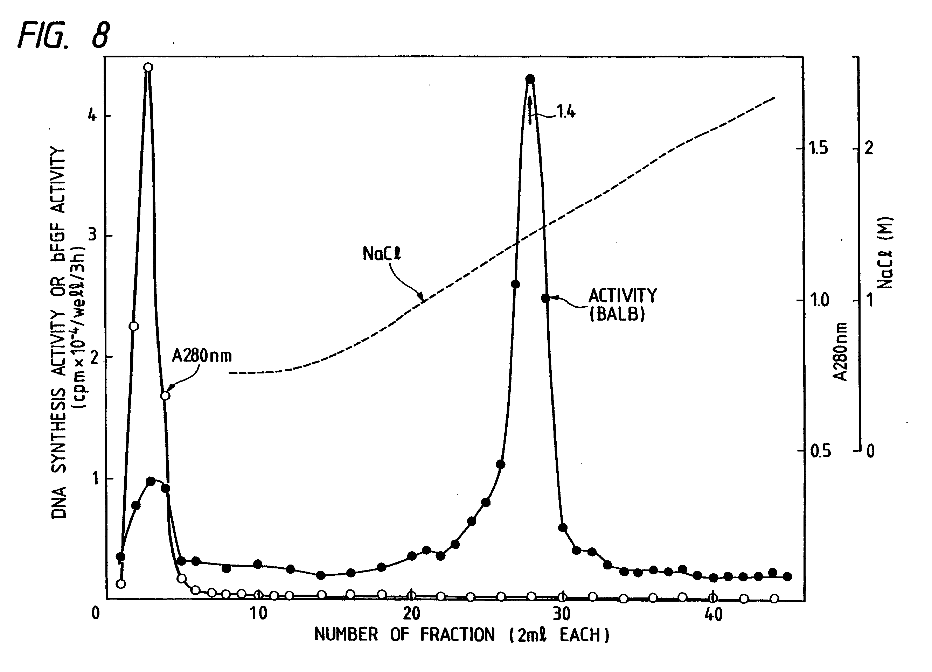

An extract from BPH tissues was subjected to Heparin5PW HPLC in the presence of 0.1% CHAPS thereto. Elution curves of FGF activity are shown in Fig. 8.

From comparison between Fig. 7 and Fig. 8, it is understood that the addition of CHAPS prevents adsorption of FGF onto a carrier and device during chromatographies.

## Claims

1. A dispersant comprising as an effective ingredient 3-[(3-cholamidopropyl)-dimethylammonio-1-propane sulfonate or 3-[(3-cholamidopropyl)-dimethylammonio]-2-hydroxy-1-propane sulfonate that is useful for stabilization of biologically active (poly)peptides.

6

EP 0 382 875 A1

*FIG. 1*

FIG. 2

EP 0 382 875 A1

FIG. 3

EP 0 382 875 A1

FIG. 4

EP 0 382 875 A1

FIG. 5

FIG. 6

FIG. 7

FIG. 8

EP 0 382 875 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 109, no. 21, 21st November 1988, pages 355-356, abstract no. 186476g, Columbus, Ohio, US; Y. MATUO et al.: "Stabilization of fibroblast growth factors by a non-cytotoxic zwitteronic detergent 3-[(3-cholamidopropyl)dimethylammonio]-1 -propane sulfonate (CHAPS)", & IN VITRO CELL. DEV. BIOL. 1988, 24(5), 477-80 * Abstract * | 1 | A 61 K 47/28 A 61 K 37/00 |
| Y | THE JOURNAL OF BIOLOGICAL CHEMISTRY, vol. 264, no. 1, 5th January 1989, pages 61-67, The American Society for Biochemistry and Molecular Biology, Inc., US; A.-L. TSAI et al.: "Solubilization of prostacyclin membrane receptors from human platelets" * Pages 66,67 * | 1 | |
| Y | CHEMICAL ABSTRACTS, vol. 101, no. 21, 19th November 1984, page 320, abstract no. 186668f, Columbus, Ohio, US; P.I. MACKENZIE et al.: "Effect of different detergent systems on the molecular size of UDP glucuronosyltransferase and other microsomal drug-metabolizing enzymes", & MEMBR. BIOCHEM. 1984, 5(3), 193-207 * Abstract * ---  -/- | 1 | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 26-10-1989 | BENZ K.F. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 109, no. 23, 5th December 1988, page 420, abstract no. 208518b, Columbus, Ohio, US; S.S. BADOUR et al.: "Detection of specific polypeptides in Chlamydomonas segnis adapted to atmospheric concentrations of carbon dioxide, using a zwitterionic detergent", & CAN. J. BOT. 1988, 66(9), 1750-4 * Abstract * | 1 | |
| Y | EP-A-0 199 992 (EISAI, CO. LTD) * Whole document * | 1 | |
| Y | EP-A-0 252 004 (CIBA-GEIGY AG) * Whole document, in particular claim 4b * | 1 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 26-10-1989 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)